# FASCICULE DE BREVET EUROPEEN

(11) **EP 4 175 541 B1**
(45) Date de publication et mention de la délivrance du brevet: **31.07.2024**
(21) Numéro de dépôt: 21737670.6
(22) Date de dépôt: 30.06.2021
(51) Int. Cl.: A61B 5/08, A61B 5/097, A61B 5/00, G01N 33/497

(54) **DISPOSITIF DE PRÉLÈVEMENT D'UNE ÉCHANTILLON GAZEUX D'HALEINE , ET PROCÉDÉ DE PRÉLÈVEMENT AU MOYEN DU DISPOSITIF DE PRÉLÈVEMENT**
VORRICHTUNG ZUM SAMMELN VON GASFÖRMIGEN ATEMPROBEN, UND SAMMELVERFAHREN MITTELS DER VORRICHTUNG
DEVICE FOR COLLECTING A GASEOUS BREATH SAMPLE, AND COLLECTION METHOD BY MEANS OF THE DEVICE

(30) Priorité: 02.07.2020 FR 2006978
(43) Date de publication de la demande: 10.05.2023
(73) Titulaire: Aryballe, 38000 Grenoble (FR)
(72) Inventeur: DUBREUIL, Romain, 38000 GRENOBLE (FR); HEWETT, Keith, BRISTOL BS16 1QY (GB); SAAD, Saliha, BRISTOL BS16 1QY (GB); ROLLAND, Guillaume, 38000 GRENOBLE (FR)
(74) Mandataire: Ipside
(86) Numéro de dépôt international: PCT/EP2021/068105
(87) Numéro de publication internationale: WO 2022/003076

(56) Documents cités:
- EP-B1- 2 968 814
- EP-B1- 3 184 485
- WO-A1-2013/189763
- CN-Y- 2 728 435
- SOPHIE BRENET ET AL: "Highly-Selective Optoelectronic Nose Based on Surface Plasmon Resonance Imaging for Sensing Volatile Organic Compounds", ANALYTICAL CHEMISTRY, vol. 90, no. 16, 19 July 2018 (2018-07-19), pages 9879 - 9887, XP055630144, ISSN: 0003-2700, DOI: 10.1021/acs.analchem.8b02036

## Description

### DOMAINE TECHNIQUE

Le domaine de l'invention est celui du prélèvement d'un échantillon gazeux d'haleine pouvant contenir des composés d'intérêt à caractériser, en particulier sur un sujet humain. L'invention vise plus particulièrement un dispositif de prélèvement comportant un embout à languette de contention, cette languette étant destinée à être introduite dans la cavité buccale d'un utilisateur pour le prélèvement d'un échantillon gazeux de son haleine. Elle trouve une application notamment dans la caractérisation de composés d'intérêt présents dans l'échantillon gazeux prélevé, au moyen par exemple d'un nez électronique utilisant une technologie d'imagerie par résonance plasmonique de surface.

### ÉTAT DE LA TECHNIQUE ANTÉRIEURE

Le document EP2906945 décrit un dispositif de prélèvement d'un échantillon d'haleine d'un sujet, pour détecter et caractériser la présence de composés cibles (ammoniaque par ex.) présents dans cet échantillon gazeux. De tels dispositifs de prélèvement d'haleine permettent de fournir des informations tirées de l'analyse d'un échantillon d'haleine et permettent par exemple l'identification et la quantification des micro-organismes présents dans la bouche du sujet. Ces dispositifs peuvent également permettre de détecter et contrôler la présence d'une substance particulière dans l'haleine du sujet. Le document précité indique que le dispositif comporte une interface de prélèvement d'haleine qui peut être constituée d'un masque ou d'un tube.

D'autres interfaces de prélèvement sont connues, comme des embouts buccaux de prélèvement d'haleine introduits dans la bouche du sujet. Un prélèvement indirect peut également être réalisé grâce à une seringue actionnée en aspiration dans la bouche du sujet, puis en transférant l'échantillon prélevé dans la seringue vers un dispositif de prélèvement d'haleine qui procède à l'analyse.

Ces embouts buccaux de prélèvement d'haleine requièrent généralement une mise en oeuvre stricte qui dépend du savoir-faire d'un praticien formé. Les résultats d'analyse sont fortement dépendants de la bonne exécution du procédé de prélèvement. Les prélèvements sont alors peu reproductibles d'une personne à l'autre. Elles peuvent également être vulnérables aux perturbations inhérentes aux conditions de prélèvement, et notamment aux conditions physicochimiques relatives à la cavité buccale du sujet, qui peuvent conduire à un bruit de mesure dégradant la qualité du prélèvement et donc de la caractérisation des composés cibles.

Par ailleurs, il existe également des embouts oropharyngés permettant d'effectuer des mesures de gaz tel que le taux de CO₂ dans l'air inspiré et expiré d'un patient alors sous anesthésie. Ces embouts comportent une ouverture principale permettant au patient de respirer lors de la mesure. Ces embouts sont décrits notamment dans les documents WO2013/ 189763A1 et CN2728435. Cependant, ils ne sont pas adaptés à effectuer un prélèvement d'haleine du patient du fait notamment de l'ouverture principale.

Enfin, le document EP2968814 B1 décrit un autre exemple d'embout de prélèvement d'un échantillon gazeux de l'haleine d'un utilisateur.

### EXPOSÉ DE L'INVENTION

L'invention a pour objectif de remédier au moins en partie aux inconvénients de l'art antérieur, et plus particulièrement de proposer un dispositif de prélèvement à embout destiné à être inséré au moins partiellement dans une cavité buccale pour y prélever un échantillon gazeux d'haleine. Le dispositif de prélèvement selon l'invention permet d'améliorer les caractéristiques du prélèvement, par exemple en termes de reproductibilité et/ou en termes de bruit de mesure La présente invention est définie par les revendications 1-13 annexées.

Pour cela, l'objet de l'invention est un dispositif de prélèvement d'un échantillon gazeux d'haleine situé dans une cavité buccale d'un utilisateur et contenant des composés cibles destinés à être caractérisés par un dispositif d'analyse, comportant :
o un embout de prélèvement de l'échantillon gazeux, adapté à obturer la cavité buccale de l'utilisateur, comportant :
   - une première face dite externe, destinée à être située hors de la cavité buccale, et au niveau de laquelle débouche un orifice externe ;
   - une deuxième face dite interne, destinée à être introduite dans la cavité buccale, opposée à la face externe suivant un axe longitudinal A_{L} de l'embout, au niveau de laquelle débouche un orifice interne relié à l'orifice externe par un conduit intérieur ;
   - une embase de positionnement, comportant la face interne, et des surfaces inférieure et supérieure de contact destinées à être au contact de dents de l'utilisateur lorsqu'il assure le maintien de l'embout dans la cavité buccale, les surfaces inférieure et supérieure étant situées de part et d'autre de l'orifice interne suivant un axe dit vertical.

Selon l'invention, l'embout comporte une première languette dite de contention, destinée à venir au contact de la langue de l'utilisateur, formée d'une plaque mince orientée de manière orthogonale à l'axe vertical et s'étendant à partir de la face interne et du côté de la surface inférieure de l'embase suivant l'axe longitudinal A_{L} sur une première longueur L_{lc} prédéfinie. De plus, le dispositif de prélèvement comporte un tuyau de prélèvement amovible, destiné à être raccordé au dispositif d'analyse et pouvant être retiré de l'embout, traversant le conduit intérieur, et s'étendant suivant l'axe longitudinal A_{L} à distance et en regard de la languette de contention sur une distance D inférieure ou égale à la première longueur L_{lc}, de sorte que la languette de contention empêche tout contact de la langue avec le tuyau de prélèvement.

Certains aspects préférés mais non limitatifs de ce dispositif de prélèvement sont les suivants.

Le dispositif de prélèvement peut comporter une deuxième languette dite de protection, formée d'une place mince orientée de manière orthogonale à l'axe vertical et s'étendant à partir de la face interne et du côté de la surface supérieure de l'embase suivant l'axe longitudinal A_{L} sur une première longueur Lₗₚ prédéfinie supérieure ou égale à la distance D à distance du tuyau de prélèvement, et agencée de manière opposée à la languette de contention suivant l'axe vertical.

La languette de contention et la languette de protection peuvent présenter des formes identiques et s'étendent symétriquement de part et d'autre de l'axe longitudinal A_{L}.

Le dispositif de prélèvement peut comporter une butée disposée sur l'embase de positionnement, destinée à être mise au contact d'une entrée de la cavité buccale.

La butée peut être formée d'une collerette saillante vis-à-vis de l'embase de positionnement.

Le dispositif de prélèvement peut comporter un appendice de préhension s'étendant dans le prolongement de l'embase de positionnement suivant l'axe longitudinal A_{L}, l'appendice de préhension comportant la face externe.

L'embase de positionnement peut présenter une forme cylindrique, définissant deux surfaces de contact, courbes et opposées l'une à l'autre suivant un axe orthogonal à l'axe longitudinal A_{L}.

Le dispositif de prélèvement peut comporter un rebord tubulaire saillant vis-à-vis de l'embase de positionnement suivant l'axe longitudinal A_{L}.

Le dispositif de prélèvement peut comporter une pluralité de conduits intérieurs recevant chacun un tuyau de prélèvement.

L'invention porte également sur un système d'analyse, comportant un dispositif de prélèvement selon l'une quelconque des caractéristiques précédentes, et un dispositif d'analyse raccordé au tuyau de prélèvement.

Le dispositif d'analyse peut comporter une chambre de mesure destinée à recevoir les composés cibles contenus dans l'échantillon gazeux, dans laquelle est située une pluralité de sites sensibles distincts comportant chacun des récepteurs aptes à interagir avec les composés cibles, le dispositif d'analyse comportant un détecteur par imagerie par résonance plasmonique de surface, ou comportant une pluralité de résonateurs électromécaniques distincts formant chacun un site sensible.

L'invention porte également sur un procédé de prélèvement au moyen du dispositif de prélèvement selon l'une quelconque des caractéristiques précédentes, dans lequel l'embout est partiellement introduit dans la cavité buccale de l'utilisateur en vue d'un prélèvement d'haleine, la languette de contention étant mise au contact de la langue du sujet. Par ailleurs, de préférence, l'utilisateur respire par le nez lors du prélèvement de l'échantillon gazeux d'haleine, ou retient sa respiration.

### BRÈVE DESCRIPTION DES DESSINS

D'autres aspects, buts, avantages et caractéristiques de l'invention apparaîtront mieux à la lecture de la description détaillée suivante de formes de réalisation préférées de celle-ci, donnée à titre d'exemple non limitatif, et faite en référence aux dessins annexés sur lesquels :
La figure 1 est une vue schématique et partielle, de côté, d'un système d'analyse comportant un dispositif d'analyse et un dispositif de prélèvement selon un mode de réalisation ;
La figure 2 est une vue schématique, en coupe longitudinale et en perspective, de l'embout de prélèvement illustré sur la figure 1 ;
La figure 3A est une vue schématique, en perspective, de l'embout de prélèvement illustré sur la figure 1 ;
La figure 3B est une vue schématique, suivant une autre perspective, de l'embout de prélèvement illustré sur la figure 1 ;
La figure 3C est une vue schématique, de face, de l'embout de prélèvement illustré sur la figure 1 ;
La figure 4 est une vue schématique de côté de l'embout de prélèvement illustré sur la figure 1, introduit dans la cavité buccale d'un utilisateur.

### EXPOSÉ DÉTAILLÉ DE MODES DE RÉALISATION PARTICULIERS

L'invention porte sur un dispositif de prélèvement d'un échantillon gazeux d'haleine pouvant contenir des composés cibles à caractériser. Ce dispositif de prélèvement comporte un embout à languette de contention et un tuyau de prélèvement amovible destiné à être raccordé à un dispositif d'analyse des composés cibles. L'embout est adapté à obturer la cavité buccale de l'utilisateur.

D'une manière générale, les composés d'intérêt (*analytes*, en anglais) sont des éléments contenus dans l'échantillon gazeux et destinés à être caractérisés par le dispositif d'analyse. Ils peuvent être, à titre illustratif, des bactéries, virus, protéines, lipides, molécules organiques volatiles, composés inorganiques, entre autres. Il peut également s'agir de composés soufrés présents dans une cavité buccale d'un sujet (personne humaine, animal...).

Le dispositif d'analyse peut être appelé 'nez électronique'. Il peut utiliser la technologie de mesure par résonance plasmonique de surface (SFR) telle que décrite par exemple dans la publication de Brenet et al. intitulé Highly-Selective Optoelectronic Nose based on Surface Plasmon résonance Imaging for Sensing Gas Phase Volatile Organic Compounds, Anal. Chem. 2018, 90, 16, 9879-9887. En variante, d'autres technologies de mesure peuvent être mises en oeuvre, telles que la mesure par résonateurs électromagnétiques de type MEMS ou NEMS (par exemple, décrit dans le document EP3184485). Plus largement, le dispositif de mesure peut être de type résistif, piézoélectrique, mécanique, acoustique ou optique.

Comme détaillé plus loin, l'embout du dispositif de prélèvement est destiné à être partiellement introduit dans la cavité buccale, et est adapté à obturer la cavité buccale de l'utilisateur. Il comporte une languette de contention destinée à venir au contact ou en vis-à-vis d'une surface qui délimite la cavité buccale, c'est-à-dire l'intérieur de la cavité buccale. Par en regard ou en vis-à-vis, on entend être situé à la perpendiculaire, suivant un axe vertical et orthogonal à l'axe longitudinal A_{L} de l'embout. L'axe est dit vertical en lien avec l'axe de serrage des dents. Le tuyau de prélèvement traverse l'embout et son extrémité libre destinée à prélever l'échantillon gazeux est alors situé en regard de la languette de contention. La longueur de la languette de contention est supérieure ou égale à la distance suivant laquelle le tuyau de prélèvement s'étend en saillie vis-à-vis d'une face interne de l'embout. Ainsi, la languette de contention permet d'éviter qu'une surface qui délimite la cavité buccale ne puisse se déplacer et venir perturber le prélèvement de l'échantillon gazeux par le tuyau de prélèvement. Ici, cette surface est notamment la langue du sujet (personne humaine, ou animal), qui, en se déplaçant, lors du prélèvement, peut modifier les conditions physicochimiques locales dans la cavité buccale (présence de gouttes de salives, taux d'humidité plus élevé, interaction mécanique avec le tuyau, etc...).

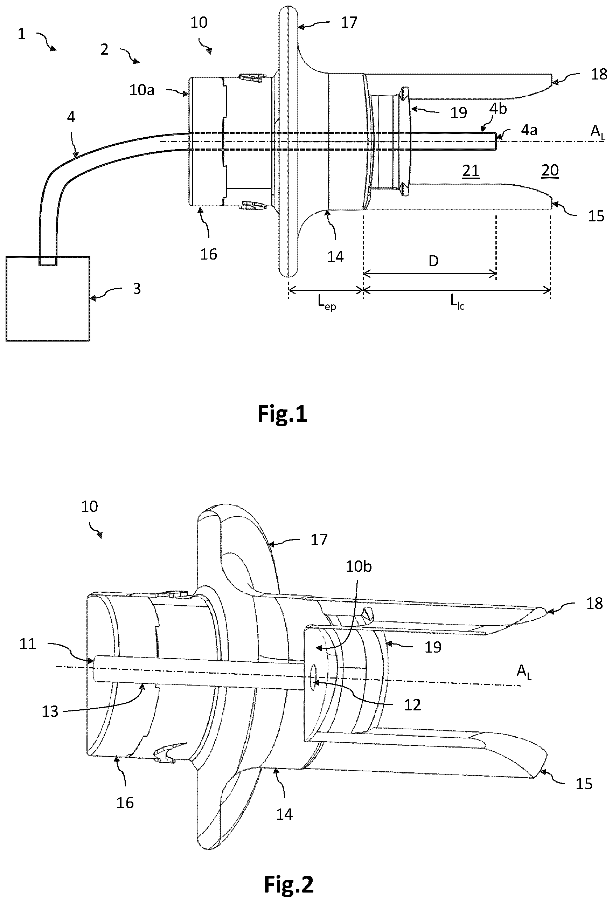

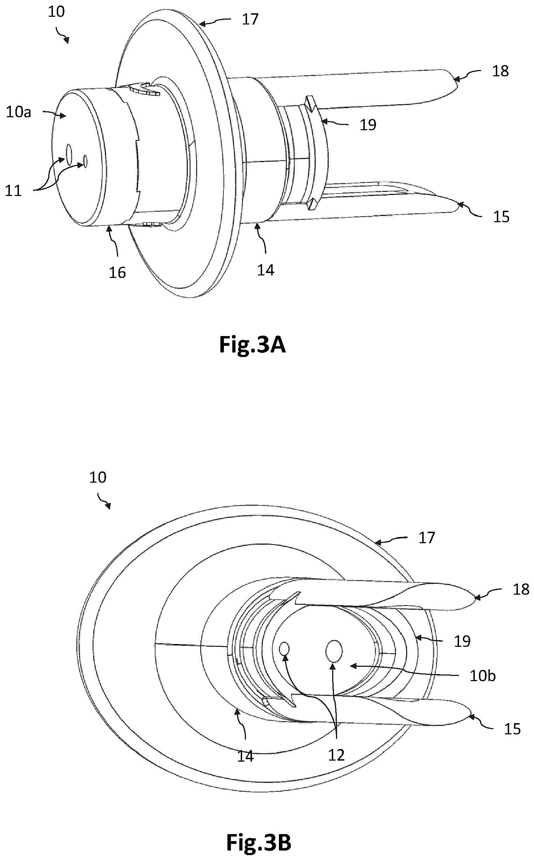

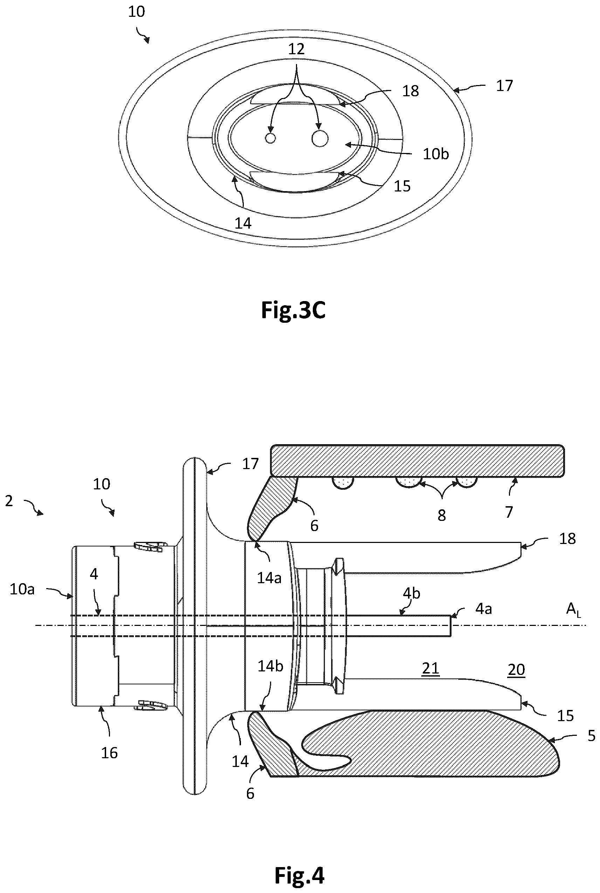

La figure 1 est une vue schématique de côté d'un système d'analyse 1 comportant un dispositif de prélèvement 2 selon un mode de réalisation, relié fluidiquement à un dispositif d'analyse 3. Les figures 2, 3A et 3B illustrent l'embout 10 suivant différentes vues. Dans cet exemple, le prélèvement est effectué dans la cavité buccale d'une personne humaine. Il s'agit en effet de prélever un échantillon gazeux de l'haleine, cet échantillon gazeux comportant des composés soufrés cibles à caractériser, situés à une distance non nulle à partir de l'entrée de la cavité buccale.

Le dispositif de prélèvement 2 comporte ainsi un embout de prélèvement 10 destiné à être en partie introduit dans la cavité buccale, lequel comporte une première languette 15 dite de contention, et un tuyau de prélèvement 4 amovible qui s'étend en regard de la languette de contention 15 et est destiné à être raccordé au dispositif d'analyse 3.

L'embout buccal 10 comporte une structure présentant :
- une première face 10a, dite face externe puisque destinée à être située hors de la cavité buccale, au niveau de laquelle débouche un premier orifice 11 dit orifice externe ; et
- une deuxième face 10b, dite face interne puisque destinée à être située dans la cavité buccale, opposée à la face externe 10a suivant un axe longitudinal A_{L} de l'embout 10, au niveau de laquelle débouche un deuxième orifice 12 dit orifice interne relié à l'orifice externe 11 par un conduit intérieur 13.

Les deux faces externe 10a et interne 10b sont ici parallèles l'une à l'autre, mais elles peuvent être inclinées l'une par rapport à l'autre. La face externe 10a comporte ici deux orifices externes 11, et la face interne 10b comporte deux orifices internes 12, deux conduits intérieurs 13 raccordant un orifice externe 11 à un orifice interne 12. En variante, les faces 10a, 10b peuvent comporter un unique orifice externe et un seul orifice interne, ou une pluralité d'orifices. Un conduit intérieur 13 raccorde de manière étanche les orifices externe 11 et interne 12. La structure de l'embout buccal 10 est réalisée en un matériau de préférence rigide et chimiquement inerte.

L'embout buccal 10 comporte une embase de positionnement 14, qui forme ici une partie de la structure de l'embout. L'embase 14 comporte la face interne 10b et est destiné à être au moins en partie introduit dans la cavité buccale. Dans cette application de prélèvement de l'haleine, elle est destinée à être maintenue dans la cavité buccale par un effort de serrage appliqué par les dents du sujet sur l'embase 14 suivant l'axe vertical. Plus précisément, elle présente une surface inférieure 14a et une surface supérieure 14b, destinées à être au contact des dents de l'utilisateur qui assure alors le maintien de l'embout buccal 10 dans la cavité buccale. Les surfaces 14a et 14b sont opposées l'une à l'autre suivant l'axe vertical (axe de serrage par les dents de l'utilisateur) et sont situées de part et d'autre de l'orifice interne 12.

Notons également que l'embout buccal 10 est adapté à obturer l'entrée de la cavité buccale, même s'il ne s'agit pas nécessairement d'une obturation étanche. Pour cela, l'embase 14 présente des dimensions transversales (dans un plan orthogonal à l'axe A_{L}) au plus égales aux dimensions de l'entrée de la cavité buccale. La collerette 17 s'étend à partir de l'embase 14 sur toute sa circonférence et présente des dimensions supérieures à l'entrée de la cavité buccale. Ainsi, lors de la mesure, l'haleine reste confinée dans la cavité buccale et n'est pas ou peu diluée par l'air extérieur entrant dans la cavité buccale.

L'embout buccal 10 comporte de préférence un appendice de préhension 16, permettant à un praticien ou au sujet lui-même de manipuler l'embout buccal 10 pour l'introduire partiellement dans la cavité buccale. L'appendice 16 comporte la face externe 10a et est destiné à ne pas être introduit dans la cavité buccale.

Dans cet exemple, l'appendice 16 et l'embase 14 sont deux parties réalisées d'un seul tenant de la même structure. En variante, il peut s'agir de deux parties assemblées l'une à l'autre mais amovibles. Ils présentent chacun une forme de cylindre à base ovale ou elliptique, disposés dans le prolongement de l'un et de l'autre suivant l'axe longitudinal A_{L} de l'embout buccal. La base cylindrique peut présenter d'autres formes, comme par exemple une forme polygonale, par exemple rectangulaire.

L'embout buccal 10 comporte une première languette 15 dite de contention. La languette de contention 15 est une plaque mince réalisée en un matériau rigide, de préférence identique à celui de la structure de l'embout 10, qui présente une longueur suivant l'axe longitudinal A_{L} supérieure à son épaisseur. Elle est orientée de manière sensiblement orthogonale à l'axe vertical, donc de manière horizontale et non pas verticale. Autrement dit, sa largeur est sensiblement orthogonale à l'axe vertical.

Elle présente une surface extérieure qui est destinée à venir au contact ou en vis-à-vis d'une surface qui délimite la cavité buccale, et une surface intérieure, opposée à la surface extérieure, qui est destinée à être en regard du tuyau de prélèvement 4. Elle s'étend sur au moins une partie de la bordure de la face interne 10b. Elle s'étend à partir de la face interne 10b suivant l'axe longitudinal A_{L} sur une longueur L_{lc} prédéfinie. Dans cet exemple, elle est positionnée en bordure de la face interne 10b du côté de la surface inférieure 14a. La languette de contention 15 est destinée à être au contact d'une surface de la cavité buccale, ici au contact de la langue, pour ainsi éviter que celle-ci ne vienne au contact de l'extrémité libre 4a du tuyau de prélèvement 4. Comme mentionné plus loin, sa longueur L_{lc} est supérieure ou égale à la distance D suivant laquelle s'étend le tuyau de prélèvement 4 le long de l'axe longitudinal A_{L} à partir de la face interne 10b. Cela permet d'améliorer la protection du tuyau de prélèvement 4 et de son extrémité libre 4a vis-à-vis de la surface de la cavité buccale, et notamment de la langue. On réduit ainsi les risques de perturbation des conditions opératoires du prélèvement, ce qui permet d'en améliorer la répétabilité et limite le bruit de mesure.

Le dispositif de prélèvement 2 comporte également un tuyau de prélèvement 4 amovible, destiné à être raccordé au dispositif d'analyse 3, et présentant une extrémité dite libre 4a destinée à être située dans la cavité buccale. Le tuyau de prélèvement 4 traverse l'embout buccal 10 par le conduit intérieur 13, et s'étend suivant l'axe longitudinal A_{L} en étant à distance et en regard de la languette de contention 15. Autrement dit, le tuyau de prélèvement 4, et plus précisément sa partie dite interne 4b qui s'étend à partir de l'orifice interne 12 suivant une direction opposée à l'orifice interne 11, s'étend de manière sensiblement parallèle à la languette de contention 15, et en est distant (pas de contact) suivant un axe dit transversal qui est orthogonal à l'axe longitudinal A_{L}. La partie interne 4b du tuyau de prélèvement 4 s'étend suivant l'axe longitudinal A_{L} sur une distance D inférieure ou égale à la longueur L_{lc} de la languette de contention 15. On évite ainsi que l'extrémité libre 4a du tuyau de prélèvement, à partir duquel l'échantillon gazeux est prélevé, ne puisse être au contact d'une surface délimitant la cavité buccale. Cette distance D peut être de l'ordre de la moitié ou de trois quarts de la longueur L_{lc}.

Le tuyau de prélèvement 4 est amovible et peut donc être inséré et retiré hors du conduit intérieur 13. Il s'étend à distance, sur toute sa longueur, vis-à-vis de la languette de contention 15 comme vis-à-vis de la languette de protection 18. Ainsi, il est possible, lors d'une série de mesure avec un même patient ou utilisateur, d'utiliser le même embout buccal 10 mais plusieurs tuyaux de prélèvement 4 : un tuyau peut être utilisé pour une mesure, puis retiré, et on insère un autre tuyau. On évite ainsi d'avoir à nettoyer l'embout 10 entre chaque mesure, et le retrait du tuyau utilisé et l'insertion d'un nouveau tuyau simplifient les mesures.

De plus, le fait que le tuyau de prélèvement 4 soit amovible dans le conduit intérieur 13 permet d'ajuster finement la position de son extrémité 4a dans la cavité buccale (c'est-à-dire sa profondeur d'enfoncement), en fonction de composants cibles que l'on souhaite caractériser. En effet, il est connu que les espèces microbiennes ne présentent pas la même concentration entre l'avant et le fond de la cavité buccale.

La figure 4 représente de manière schématique et partielle, de côté, l'embout buccal 10 illustré sur la figure 1 introduit partiellement dans une cavité buccale. Celle-ci est représentée en coupe longitudinale, et la langue 5, les dents 6 et le palais 7 sont représentés schématiquement.

Dans le but ici de permettre au sujet de maintenir l'embout buccal 10 avec ses dents 6, l'embase 14 présentant deux surfaces de contact 14a, 14b, opposées l'une à l'autre vis-à-vis de l'axe vertical et agencées de part et d'autre de l'orifice interne 12. Ces surfaces de contact 14a, 14b sont destinées à recevoir les dents 6 du sujet, qui applique alors un effort de serrage l'une vers l'autre.

L'embout buccal 10 comporte avantageusement une butée 17, ici sous la forme d'une collerette, disposée sur l'embase 14 et adjacente à l'appendice 16, qui peut constituer une référence de positionnement vis-à-vis de l'entrée de la cavité buccale. La butée 17 est ici une paroi qui s'étend de manière transversale à l'axe longitudinal A_{L}, sur au moins une partie de la périphérie de l'embase 14. Dans cet exemple, la butée 17 est destinée à venir se plaquer contre les lèvres du sujet (non représentées) lorsqu'il mord l'embase 14, de sorte que l'appendice 16 et la butée 17 restent en dehors de la cavité buccale. Elle participe à obturer l'entrée de la cavité buccale.

De préférence, l'embout buccal 10 comporte une deuxième languette 18, dite languette de protection, qui est ici sensiblement symétrique de la languette de contention 15 vis-à-vis de l'axe longitudinal A_{L} (elle est donc opposée à la languette 15 suivant l'axe vertical). Elle est une plaque mince réalisée en un matériau rigide, de préférence identique à celui de la structure de l'embout 10, qui présente une longueur suivant l'axe longitudinal A_{L} supérieure à son épaisseur. Elle présente une surface extérieure qui est destinée à venir au contact ou en vis-à-vis d'une surface qui délimite la cavité buccale, et une surface intérieure, opposée à la surface extérieure, qui est destinée à être en regard du tuyau de prélèvement 4 et de la surface intérieure de la languette de contention 15. Elle est orientée de manière orthogonale à l'axe vertical, et n'est donc pas horizontale.

Elle s'étend sur au moins une autre partie de la bordure de la face interne 10b, du côté de la surface supérieure 14b. Elle s'étend également à partir de la face interne 10b suivant l'axe longitudinal A_{L} sur une longueur Lₗₚ prédéfinie. Dans cet exemple, elle est également positionnée en bordure de la face interne 10b. La languette de protection 18 est destinée à être au moins en vis-à-vis d'une surface de la cavité buccale, ici en vis-à-vis du palais, pour ainsi éviter que d'éventuelles gouttes 8 ne tombent sur la partie interne 4b du tuyau de prélèvement 4, et plus précisément ne tombent sur l'extrémité libre 4a. Elle s'étend donc à distance du tuyau de prélèvement 4. De telles gouttes pourraient ainsi augmenter le taux d'humidité de l'échantillon gazeux prélevé, ce qui peut induire un bruit de mesure lors de la caractérisation des composés cibles. Sa longueur Lₗₚ est supérieure ou égale à la distance D suivant laquelle s'étend le tuyau de prélèvement 4 le long de l'axe longitudinal A_{L} à partir de la face interne 10b.

Les languettes de contention 16 et de protection 18 sont ici distinctes l'une de l'autre. Elles délimitent un espace interne dans lequel est située la partie interne 4b du tuyau de prélèvement 4. Cet espace interne présente une ouverture de fond 20 orientée vers l'axe longitudinal A_{L} et par lequel l'échantillon gazeux est aspiré. Il présente ici deux ouvertures latérales 21, opposées l'une à l'autre suivant un axe transversal. En variante, les deux languettes peuvent se rejoindre latéralement par l'intermédiaire de parois latérales, et former ainsi une même paroi périphérique qui s'étend continûment autour de la partie interne 4b du tuyau de prélèvement 4. Dans ce cas, cette paroi périphérique de contention et de protection présente avantageusement plusieurs orifices traversants (rainurage, perforation...), agencés au niveau d'un axe transversal dit horizontal, c'est-à-dire orthogonal à l'axe longitudinal A_{L} et à l'axe vertical (lequel est orienté de la langue 5 vers le palais 7).

Comme mentionné précédemment, l'embout buccal 10 comporte au moins un conduit intérieur 13, avec orifices externe 11 et interne 12 correspondants, pour permettre l'insertion du tuyau de prélèvement 4 et donc le prélèvement de l'échantillon gazeux d'haleine. Dans le présent exemple, l'embout buccal 10 comporte deux conduits intérieurs 13 permettant d'insérer deux tuyaux et donc de réaliser deux prélèvements d'haleine simultanés. En variante, l'embout buccal 10 comporte un seul conduit intérieur 13, ou autant de conduits intérieurs 13 que de prélèvements simultanés souhaités. Les tuyaux peuvent présenter des profondeurs d'enfoncement différentes, pour mesurer différents composés cibles ou un gradient de concentration d'un même composé cible. Un conduit interne 13 présente des dimensions adaptées à celles du tuyau de prélèvement 4 pour permettre l'insertion et le retrait du tuyau tout en limitant les échanges fluidiques entre l'air extérieur et l'haleine située dans la cavité buccale.

L'embout buccal comporte ici un rebord tubulaire 19 prolongeant le profil cylindrique de l'embase 14 et s'étendant donc en saillie vis-à-vis de l'embase 14 suivant l'axe longitudinal A_{L}, les orifices internes 12 débouchant dans l'espace délimité par ce rebord tubulaire 19.

L'embout buccal 10 est de préférence réalisé d'une seule pièce, par exemple par moulage par injection dans une résine adaptée aux procédés de stérilisation courants. L'embase 14 est pleine (sans ouverture principale, si ce n'est le ou les conduits internes pour assurer le passage du ou des tuyaux de prélèvement). Sa constitution avec une languette de contention 15 et une languette de protection 18 identiques l'une à l'autre, s'étendant symétriquement le long de l'axe longitudinal A_{L}, simplifie l'utilisation en réduisant les consignes à la seule directive de mettre l'un des deux éléments contre la langue du sujet.

Un exemple de procédé de prélèvement est maintenant décrit dans le cadre d'un prélèvement d'haleine chez un sujet, au moyen du dispositif de prélèvement décrit en référence à la fig.1.

L'embout buccal 10 est tout d'abord stérilisé par tout procédé connu, par exemple à la vapeur dans un dispositif autoclave. Il est ensuite éventuellement nettoyé pour éliminer toute trace de produit de stérilisation qui pourrait interférer avec le prélèvement d'haleine.

Selon un mode de réalisation particulièrement avantageux, illustré sur les figures 1 et 4, l'embout buccal 10 est tout d'abord équipé d'au moins un tuyau de prélèvement 4 amovible et jetable. Le tuyau de prélèvement 4 est inséré dans le conduit intérieur 13 et traverse ainsi l'embase 14 et l'appendice 16, en étant saillant d'une part par l'orifice externe 11 et d'autre part par l'orifice interne 12. À partir de l'orifice interne 12, le tuyau 4 s'étend suivant l'axe longitudinal A_{L} jusqu'à une distance calibrée D définie entre la face interne 10b et l'extrémité libre 4a du tuyau 4.

L'extrémité opposée du tuyau de prélèvement 4 est raccordée à un dispositif d'analyse 3 adapté à réaliser le prélèvement par aspiration, la détection et la caractérisation des composés cibles présents dans l'échantillon gazeux.

Une fois le tuyau de prélèvement 4 en place, l'appendice de préhension 14 est saisi par le sujet, et l'embout buccal 10 est introduit dans la bouche, de sorte que la languette de contention 15 vienne contre la langue 5 du sujet qui serre ensuite ses dents 6 sur l'embase 14 en mettant ses lèvres au contact de la butée 17. L'entrée de la cavité buccale est alors obturée. Le dispositif d'analyse 3 est ensuite activé pour réaliser le prélèvement d'haleine par aspiration puis la caractérisation des composés cibles.

Durant le prélèvement d'haleine, l'échantillon gazeux est aspiré par l'extrémité libre 4a du tuyau de prélèvement 4, c'est-à-dire à une distance longitudinale D calibrée à l'intérieur de la bouche du sujet. Cette distance D est choisie en fonction de la zone précise de prélèvement souhaité, qui peut exiger un échantillonnage, par exemple, plutôt à proximité des dents ou plutôt vers le fond de la cavité buccale. Le prélèvement est de préférence effectué vers le fond de la cavité buccale dans la mesure où il apparaît que la concentration en composés soufrés à caractériser y est plus élevée. Notons que lors du prélèvement, le sujet respire par le nez et ne peut respirer par la bouche : l'embout 10 obture l'entrée de la cavité buccale, et les dimensions du conduit intérieur 13 comme celles du tuyau 4 sont insuffisantes pour permettre au sujet de respirer par la bouche ou de permettre un échange fluidique entre l'haleine et l'air extérieur. On évite ainsi que l'haleine ne soit diluée dans le flux d'air pulmonaire, de sorte que la concentration des composés cibles à caractériser n'est pas dégradée.

Durant le prélèvement, la languette de contention 15 empêche la mise en contact de la langue 5 du sujet avec le tuyau de prélèvement 4 et empêche ainsi la salive du sujet de venir perturber le prélèvement d'haleine, par exemple en introduisant dans l'haleine prélevée de la vapeur issue de l'évaporation de salive à proximité de l'extrémité libre 4a. De même, la languette de protection 18 protège le tuyau de prélèvement 4, et son extrémité libre 4a, de la chute de gouttelettes 8 de salive, par exemple, issues du palais 7 du sujet, pour ne pas perturber le prélèvement d'haleine.

Lorsque l'embout buccal 10 comporte par exemple deux conduits intérieurs 13 qui sont équipés de deux tuyaux de prélèvement 4, les conduits intérieurs 13 peuvent présenter des sections différentes, et logent donc des tuyaux de prélèvement 4 de sections ou diamètres différents. Ces deux tuyaux de prélèvement 4 peuvent être reliés au même dispositif d'analyse, ou à deux dispositifs d'analyse différents réalisant chacun une analyse différente de son prélèvement respectif. Ils peuvent présenter des profondeurs d'enfoncement différentes.

Deux prélèvements d'haleine simultanés peuvent être réalisés avec leur section respective pour les tuyaux de prélèvement 4 ainsi que leur distance D calibrée respective. Deux prélèvements simultanés avec des tuyaux de prélèvement 4 positionnés selon deux distances calibrées D différentes permettent de réaliser deux prélèvements à deux profondeurs différentes dans la cavité buccale.

Des variantes de réalisation de l'embout buccal 10 peuvent être mises en oeuvre sans sortir du cadre de l'invention.

Ainsi, l'embase 14 peut comporter une gaine en saillie vis-à-vis de la face interne 10b suivant l'axe A_{L} et débouchant sur l'orifice interne 12, assurant un maintien mécanique supplémentaire de la partie interne 4b du tuyau de prélèvement 4.

Par ailleurs, la butée 17 peut par ailleurs être prévue pour venir directement contre les dents du sujet plutôt que contre ses lèvres. La butée 17 peut alors être formée par une gorge pratiquée dans l'embase 14, ou par une collerette de diamètre adapté.

## Revendications

1. Dispositif de prélèvement (2) d'un échantillon gazeux d'haleine d'un utilisateur et contenant des composés cibles destinés à être **caractérisés par** un dispositif d'analyse (3), comportant :
o un embout (10) de prélèvement de l'échantillon gazeux, adapté à obturer la cavité buccale de l'utilisateur, et comportant :
• une première face (10a) dite externe, destinée à être située hors de la cavité buccale, et au niveau de laquelle débouche un orifice externe (11) ;
• une deuxième face (10b) dite interne, destinée à être introduite dans la cavité buccale, opposée à la face externe (10a) suivant un axe longitudinal A_{L} de l'embout (10), au niveau de laquelle débouche un orifice interne (12) relié à l'orifice externe (11) par un conduit intérieur (13) ;
• une embase (14) de positionnement, comportant la face interne (10b), et des surfaces inférieure et supérieure de contact (14a, 14b) destinées à être au contact de dents de l'utilisateur lorsqu'il assure le maintien de l'embout dans la cavité buccale, les surfaces inférieure et supérieure étant situées de part et d'autre de l'orifice interne (12) suivant un axe dit vertical ;
o **caractérisé en ce que** l'embout (10) comporte une première languette (15) dite de contention, destinée à venir au contact de la langue de l'utilisateur, formée d'une plaque mince orientée de manière orthogonale à l'axe vertical et s'étendant à partir de la face interne (10b) et du côté de la surface inférieure (14b) de l'embase (14) suivant l'axe longitudinal A_{L} sur une première longueur L_{lc} prédéfinie ;
o et **en ce que** le dispositif de prélèvement (2) comporte un tuyau de prélèvement (4) amovible, destiné à être raccordé au dispositif d'analyse (3) et pouvant être retiré de l'embout (10), traversant le conduit intérieur (13), et s'étendant suivant l'axe longitudinal A_{L} à distance et en regard de la languette de contention (15) sur une distance D inférieure ou égale à la première longueur L_{lc}, de sorte que la languette de contention (15) empêche tout contact de la langue avec le tuyau de prélèvement (4).

2. Dispositif de prélèvement (2) selon la revendication 1, comportant une deuxième languette (18) dite de protection, formée d'une place mince orientée de manière orthogonale à l'axe vertical et s'étendant à partir de la face interne (10b) et du côté de la surface supérieure (14a) de l'embase (14) suivant l'axe longitudinal A_{L} sur une première longueur Lₗₚ prédéfinie supérieure ou égale à la distance D, à distance du tuyau de prélèvement (4), et agencée de manière opposée à la languette de contention (15) suivant l'axe vertical.

3. Dispositif de prélèvement (2) selon la revendication 2, dans lequel la languette de contention (15) et la languette de protection (18) présentent des formes identiques et s'étendent symétriquement de part et d'autre de l'axe longitudinal A_{L}.

4. Dispositif de prélèvement (2) selon l'une quelconque des revendications 1 à 3, comportant une butée (17) disposée sur l'embase (14) de positionnement, destinée à être mise au contact d'une entrée de la cavité buccale.

5. Dispositif de prélèvement (2) selon la revendication 4, dans lequel la butée (17) est formée d'une collerette saillante vis-à-vis de l'embase (14) de positionnement.

6. Dispositif de prélèvement (2) selon l'une quelconque des revendications 1 à 5, comportant un appendice de préhension (16) s'étendant dans le prolongement de l'embase (14) de positionnement suivant l'axe longitudinal A_{L}, l'appendice de préhension (16) comportant la face externe (10a).

7. Dispositif de prélèvement (2) selon l'une quelconque des revendications précédentes, **caractérisé en ce que** l'embase (14) de positionnement présente une forme cylindrique, définissant deux surfaces de contact, courbes et opposées l'une à l'autre suivant un axe orthogonal à l'axe longitudinal A_{L}.

8. Dispositif de prélèvement (2) selon l'une quelconque des revendications précédentes, comportant un rebord tubulaire (19) saillant vis-à-vis de l'embase (14) de positionnement suivant l'axe longitudinal A_{L}.

9. Dispositif de prélèvement (2) selon l'une quelconque des revendications précédentes, comportant une pluralité de conduits intérieurs (13) recevant chacun un tuyau de prélèvement (4).

10. Système d'analyse (1), comportant un dispositif de prélèvement (2) selon l'une quelconque des revendications précédentes, et un dispositif d'analyse (3) raccordé au tuyau de prélèvement (4).

11. Système d'analyse (1) selon la revendication 10, le dispositif d'analyse comportant une chambre de mesure destinée à recevoir les composés cibles contenus dans l'échantillon gazeux, dans laquelle est située une pluralité de sites sensibles distincts comportant chacun des récepteurs aptes à interagir avec les composés cibles, le dispositif d'analyse comportant un détecteur par imagerie par résonance plasmonique de surface, ou comportant une pluralité de résonateurs électromécaniques distincts formant chacun un site sensible.

12. Procédé de prélèvement au moyen du dispositif de prélèvement (2) selon l'une quelconque des revendications 1 à 9, dans lequel l'embout (10) est partiellement introduit dans la cavité buccale de l'utilisateur en vue d'un prélèvement d'haleine, la languette de contention (15) étant mise au contact de la langue (5) du sujet.

13. Procédé de prélèvement selon la revendication 12, dans lequel, lors du prélèvement de l'échantillon gazeux d'haleine, l'utilisateur respire par le nez.

## Patentansprüche

1. Vorrichtung (2) zum Entnehmen einer gasförmigen Atemprobe von einem Benutzer und einer enthaltenden Zielverbindung, die dazu bestimmt ist, von einer Analysevorrichtung (3) charakterisiert zu werden, enthaltend:
o ein Mundstück (10) zum Entnehmen der gasförmigen Probe, das dazu ausgelegt ist, die Mundhöhle des Benutzers zu verschließen, und Folgendes umfasst:
• eine erste, Außenfläche genannte, Fläche (10a), die dazu bestimmt ist, sich außerhalb der Mundhöhle zu befinden, und an der eine äußere Öffnung (11) mündet;
• eine zweite, Innenfläche genannte, Fläche (10b), die dazu bestimmt ist, in die Mundhöhle eingeführt zu werden, die der Außenfläche (10a) entlang einer Längsachse A_{L} des Mundstücks (10) gegenüberliegt und an der eine innere Öffnung (12) mündet, die durch einen inneren Kanal (13) mit der äußeren Öffnung (11) verbunden ist;
• eine Positionierungsbasis (14), die die innere Fläche (10b) und eine obere und eine untere Kontaktfläche (14a, 14b) umfasst, die dazu bestimmt sind, mit den Zähnen des Benutzers in Kontakt zu stehen, wenn er sicherstellt, dass das Mundstück in der Mundhöhle gehalten wird, wobei sich die untere und die obere Kontaktfläche auf beiden Seiten der inneren Öffnung (12) entlang einer vertikalen Achse befinden;
o **dadurch gekennzeichnet, dass** das Mundstück (10) eine erste Haltelasche (15) umfasst, die dazu bestimmt ist, in Kontakt mit der Zunge des Benutzers zu kommen, die aus einer dünnen Platte gebildet ist, die orthogonal zur vertikalen Achse ausgerichtet ist und sich von der inneren Fläche (10b) und der Seite der unteren Kontaktfläche (14b) der Basis (14) entlang der Längsachse A_{L} über eine erste vordefinierte Länge L_{lc} erstreckt;
o und dadurch, dass die Entnahmevorrichtung (2) ein abnehmbares Entnahmerohr (4) umfasst, das dazu bestimmt ist, mit der Analysevorrichtung (3) verbunden zu werden, und das von dem Mundstück (10) abgenommen werden kann, das durch den inneren Kanal (13) verläuft und sich entlang der Längsachse A_{L} und gegenüber der Haltelasche (15) über eine Distanz D erstreckt, die kleiner oder gleich der ersten Länge L_{lc} ist, so dass die Haltelasche (15) jeglichen Kontakt der Zunge des Benutzers mit dem Entnahmerohr (4) verhindert.

2. Entnahmevorrichtung (2) nach Anspruch 1, die eine zweite Schutzlasche (18) umfasst, die aus einer dünnen Platte gebildet ist, die orthogonal zur vertikalen Achse ausgerichtet ist und sich von der inneren Fläche (10b) und der Seite der oberen Fläche (14a) der Basis (14) entlang der Längsachse A_{L} über eine erste vordefinierte Länge Lₗₚ erstreckt, die größer oder gleich der Distanz D ist, von dem abnehmbaren Entnahmerohr (4) beabstandet und gegenüber der Haltelasche (15) entlang der vertikalen Achse angeordnet ist.

3. Entnahmevorrichtung (2) nach Anspruch 2, wobei die Haltelasche (15) und die Schutzlasche (18) identische Formen aufweisen und sich symmetrisch auf beiden Seiten der Längsachse A_{L} erstrecken.

4. Entnahmevorrichtung (2) nach einem der Ansprüche 1 bis 3, die einen Anschlag (17) umfasst, der auf der Positionierungsbasis (14) angeordnet und dazu bestimmt ist, mit einem Eingang der Mundhöhle in Kontakt zu gelangen.

5. Entnahmevorrichtung (2) nach Anspruch 4, wobei der Anschlag (17) aus einem vorstehenden Flansch gebildet ist, der der Positionierungsbasis (14) zugewandt ist.

6. Entnahmevorrichtung (2) nach einem der Ansprüche 1 bis 5, die einen Greiffortsatz (16) umfasst, der sich in einer Verlängerung der Positionierungsbasis (14) entlang der Längsachse A_{L} erstreckt, wobei der Greiffortsatz (16) die äußere Fläche (10a) umfasst.

7. Entnahmevorrichtung (2) nach einem der vorhergehenden Ansprüche, **dadurch gekennzeichnet, dass** die Positionierungsbasis (14) eine zylindrische Form aufweist, die zwei Kontaktflächen definiert, die gekrümmt sind und einander entlang einer zur Längsachse A_{L} orthogonal liegenden Achse gegenüberliegen.

8. Entnahmevorrichtung (2) nach einem der vorhergehenden Ansprüche, die einen vorstehenden rohrförmigen Rand (19) umfasst, der der Positionierungsbasis (14) entlang der Längsachse A_{L} zugewandt ist.

9. Entnahmevorrichtung (2) nach einem der vorhergehenden Ansprüche, die eine Vielzahl von inneren Kanälen (13) umfasst, die jeweils ein Entnahmerohr (4) aufnehmen.

10. Analysesystem (1), das eine Entnahmevorrichtung (2) nach einem der vorhergehenden Ansprüche und eine Analysevorrichtung (3) umfasst, die mit dem Entnahmerohr (4) verbunden ist.

11. Analysesystem (1) nach Anspruch 10, wobei die Analysevorrichtung eine Messkammer umfasst, die dazu bestimmt ist, die in der gasförmigen Probe enthaltenen Zielverbindungen aufzunehmen, in der sich eine Vielzahl von verschiedenen empfindlichen Stellen befindet, die jeweils Empfänger umfassen, der dazu geeignet sind, mit den Zielverbindungen zu interagieren, wobei die Analysevorrichtung einen Oberflächenplasmonenresonanz-Bildgebungsdetektor oder eine Vielzahl von separaten elektromechanischen Resonatoren umfasst, die jeweils eine empfindliche Stelle bilden.

12. Entnahmeverfahren mittels der Entnahmevorrichtung (2) nach einem der Ansprüche 1 bis 9, wobei das Mundstück (10) teilweise in die Mundhöhle des Benutzers hinsichtlich einer Atementnahme eingeführt wird, wobei die Haltelasche (15) in Kontakt mit der Zunge (5) des Patienten gebracht wird.

13. Entnahmeverfahren nach Anspruch 12, wobei der Benutzer während des Entnehmens der gasförmigen Atemprobe durch die Nase atmet.

## Claims

1. Collection device (2) for collecting a breath sample from a user and containing target compounds to be **characterized by** an analysis device (3), including:
o a mouthpiece (10) for collecting the breath sample, configured to fully close the oral cavity of the user, and including:
• a first face (10a), referred to as the external face, intended to be located outside the oral cavity and at which an external orifice (11) opens out:
• a second face (10b), referred to as the internal face, intended to be inserted into the oral cavity, opposite the external face (10a) along a longitudinal axis A_{L} of the mouthpiece (10) at which an internal orifice (12) opens out which is connected to the external orifice (11) by an internal duct (13);
• a positioning base (14), including the internal face (10b), and lower and upper contact surfaces (14a, 14b) which are intended to be in contact with users' teeth when they hold the mouthpiece in the oral cavity, the lower and upper surfaces being located on either side of the internal orifice (12) along an axis referred to as the vertical axis;
o **characterized in that** the mouthpiece (10) includes a first tab (15), referred to as a retaining tab, which is configured to come into contact with the user's tongue and is formed by a thin plate oriented orthogonally to the vertical axis and extending from the internal face (10b) and from the side of the lower surface (14b) of the base (14) along the longitudinal axis A_{L} over a first predefined length L_{lc};
o and **in that** the collection device (2) includes a removable sampling tube (4), configured to be connected to the analysis device (3) and able to be removed from the mouthpiece (10), passing through the internal duct (13), and extending along the longitudinal axis A_{L} away from and opposite the retaining tab (15) over a distance D that is less than or equal to the first length L_{lc}, so that the retaining tab (15) prevents any contact of the tongue with the sampling tube (4).

2. Collection device (2) according to claim 1, including a second tab (18), referred to as a protection tab, formed by a thin plate oriented orthogonally to the vertical axis and extending from the internal face (10b) and from the side of the upper surface (14a) of the base (14) along the longitudinal axis A_{L} over a first predefined length Lₗₚ that is greater than or equal to the distance D, at a distance from the sampling tube (4), and arranged opposite the retaining tab (15) along the vertical axis.

3. Collection device (2) according to claim 2, wherein the retaining tab (15) and the protection tab (18) have an identical shape and extend symmetrically on either side of the longitudinal axis A_{L}.

4. Collection device (2) according to any one of claims 1 to 3, including a stop (17) arranged on the positioning base (14), intended to be brought into contact with an entrance of the oral cavity.

5. Collection device (2) according to claim 4, wherein the stop (17) is formed by a collar projecting from the positioning base (14).

6. Collection device (2) according to any one of claims 1 to 5, including a gripping appendage (16) extending in extension of the positioning base (14) along the longitudinal axis A_{L}, the gripping appendage (16) including the external face (10a).

7. Collection device (2) according to any one of the preceding claims, **characterized in that** the positioning base (14) has a cylindrical shape, defining two contact surfaces that are curved and opposite one another along an axis orthogonal to the longitudinal axis A_{L}.

8. Collection device (2) according to any one of the preceding claims, including a tubular rim (19) protruding from the positioning base (14) along the longitudinal axis A_{L}.

9. Collection device (2) according to any one of the preceding claims, including a plurality of internal ducts (13) each receiving a sampling tube (4).

10. Analysis system (1), including a sampling device (2) according to any one of the preceding claims and an analysis device (3) connected to the sampling tube (4).

11. Analysis system (1) according to claim 10, the analysis device including a measuring chamber configured to receive target compounds contained in the breath sample, in which a plurality of distinct sensitive sites are located, each including receivers configured to interact with the target compounds, the analysis device including a surface plasmon resonance imaging detector or including a plurality of separate electromechanical resonators each forming a sensitive site.

12. Method of sampling by means of the collection device (2) according to any one of claims 1 to 9, wherein the mouthpiece (10) is partially inserted into the user's oral cavity for taking a breath sample, the retaining tab (15) being brought into contact with the subject's tongue (5).

13. Sampling method according to claim 12, wherein, during the collection of the breath sample, the user breathes through the nose.
